# EUROPEAN PATENT APPLICATION

(11) **EP 4 848 205 A2**
(43) Date of publication of application: **02.12.2026**
(21) Application number: 26211545.4
(22) Date of filing: 21.03.2023
(51) Int. Cl.: G06T 7/10

(54) **SYSTEMS FOR PLANNING AND PROVIDING NAVIGATION GUIDANCE FOR ANATOMIC LUMEN TREATMENT**

(30) Priority: 24.03.2022 US 202263323457 P; 03.08.2022 US 202263394871 P; 03.08.2022 US 202263394873 P
(62) Document number(s) of the earlier application(s) in accordance with Art. 76 EPC: 23717378.6 / 4 498 963
(71) Applicant: Intuitive Surgical Operations, Inc., Sunnyvale, CA 94086 (US)
(72) Inventor: WONG, Serena H., Sunnyvale, 94086 (US); ADEBAR, Troy K., Sunnyvale, 94086 (US); BARBAGLI, Federico, Sunnyvale, 94086 (US); ZHANG, Hui, Sunnyvale, 94086 (US)
(74) Representative: J A Kemp LLP

(57) **Abstract**

A system comprising: a processor; and a memory having computer readable instructions stored thereon, the computer readable instructions, when executed by the processor, cause the system to: identify a plurality of passageways in an anatomic region; identify at least one area of anatomic constraint in the plurality of passageways; and determine an ablation treatment plan based on the at least one area of anatomic constraint, the ablation treatment plan including a treatment order for the passageways and a plurality of sequential treatment locations.

## Description

### CROSS-REFERENCED APPLICATIONS

This application claims priority to and the benefit of U.S. Provisional Application No. 63/323,457, filed March 24, 2022, entitled "Systems and Methods for the Planning, Navigation, and/or Delivery of an Anatomic Lumen Treatment," U.S. Provisional Application No. 63/394,871, filed August 3, 2022, entitled "Systems and Methods for the Planning, Navigation, and/or Delivery of an Anatomic Lumen Treatment," and U.S. Provisional Application No. 63/394,873, filed August 3, 2022, entitled "Systems and Methods for Planning and Providing Navigation Guidance for Anatomic Lumen Treatment," which are incorporated by reference herein in their entirety.

### FIELD

Examples described herein relate to systems and methods for endoluminal treatment. More particularly, examples may relate to planning, navigation, and delivery of ablation treatment within an anatomic lumen.

### BACKGROUND

Minimally invasive medical techniques may generally be intended to reduce the amount of tissue that is damaged during medical procedures, thereby reducing patient recovery time, discomfort, and harmful side effects. Such minimally invasive techniques may be performed through natural orifices in a patient anatomy or through one or more surgical incisions. Through these natural orifices or incisions an operator may insert minimally invasive medical instruments such as therapeutic instruments, diagnostic instruments, imaging instruments, and surgical instruments. Some minimally invasive medical instruments may be used to perform ablation. Improved systems and methods for planning ablation treatment, navigating instruments to the treatment area, and delivering ablation treatment are needed to control the treated luminal surface area, achieve good contact between the instrument and the lumen wall, minimize ablation lesion overlap.

### SUMMARY

The embodiments of the invention are best summarized by the claims that follow the description.

In one example, a system may comprise a processor and a memory having computer readable instructions stored thereon. The computer readable instructions, when executed by the processor, may cause the system to receive anatomic image data for an anatomic area and segment the anatomic image data to identify an anatomic passageway in the anatomic area. The computer readable instructions may also cause the system to identify a gap between a first segment and a second segment of the segmented anatomic image data, generate a bridge segment to bridge the gap, and generate an anatomic model of a diseased lung including the first segment, the second segment and the bridge segment.

In another example, a system may comprise a processor and a memory having computer readable instructions stored thereon. The computer readable instructions, when executed by the processor, may cause the system to generate an ablation treatment plan, provide navigation guidance to perform an ablation treatment according to the ablation treatment plan, and generate a treatment report after performance of the ablation treatment according to the ablation treatment plan.

In another example, a system may comprise a robot-assisted manipulator, an ablation device configured for coupling to the robotic-assisted manipulator, and a control system coupled to the robot-assisted manipulator. The control system may be configured to determine a first ablation treatment according to a first treatment mode for a first endoluminal location, receive anatomic information about a second endoluminal location, and, based on the anatomic information, determine a second ablation treatment according to a second treatment mode for the second endoluminal location.

In another example, a system may comprise a robot-assisted manipulator, an elongate instrument configured for manipulation by the robot-assisted manipulator, an ablation device coupled to a distal portion of the elongate instrument, a sensor system coupled to the elongate instrument, and a control system coupled to the robot-assisted manipulator. The control system may be configured to actuate the robot-assisted manipulator to move the ablation device from a first endoluminal location in an anatomic passageway to a second endoluminal location in the anatomic passageway. An expected rate of ablation device motion may be associated with the actuation of the robot-assisted manipulator. The control system is also configured to receive sensor data from the sensor system while actuating the robot-assisted manipulator, deliver an ablation treatment to the anatomic passageway while actuating the robot-assisted manipulator, and based on the received sensor data and the expected rate of ablation device motion, evaluate ablation device contact with the anatomic passageway.

It is to be understood that both the foregoing general description and the following detailed description are illustrative and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTIONS OF THE DRAWINGS

FIG. 1A is a simplified diagram of a patient anatomy, according to some examples.
FIG. 1B illustrates a detailed portion of the diagram of FIG. 1A.
FIG. 2 is a flowchart illustrating a method for generating an anatomic model.
FIG. 3 is a flowchart illustrating a method for performing a treatment, such as an ablation treatment.
FIG. 4 is a schematic illustration of parameters for a treatment plan.
FIG. 5 illustrates a graphical user interface for providing navigational guidance.
FIG. 6 illustrates a graphical user interface for providing navigational guidance.
FIG. 7 is a flowchart illustrating a method for using anatomic information to determine a treatment mode for an ablation device.
FIG. 8 is a flowchart illustrating a method for evaluating the efficacy of an ablation procedure.
FIG. 9 is a robot-assisted medical system, according to some examples.

Examples of the present disclosure and their advantages are best understood by referring to the detailed description that follows. It should be appreciated that like reference numerals are used to identify like elements illustrated in one or more of the figures, wherein showings therein are for purposes of illustrating examples of the present disclosure and not for purposes of limiting the same.

### DETAILED DESCRIPTION

The technology described herein provides techniques and treatment systems for luminal treatment of diseased tissue. Although the examples provided herein may refer to treatment of lung tissue and pulmonary disease, it is understood that the described technology may be used in treating artificially created lumens or any endoluminal or vascular passageway or cavity, including in a patient trachea, colon, intestines, stomach, liver, kidneys and kidney calices, brain, heart, circulatory system including vasculature, fistulas, and/or the like. In some examples, treatment described herein may be used in procedures to treat lung tumors, asthma, and/or chronic obstructive pulmonary disease (COPD) that may include one or more of a plurality of disease conditions including chronic bronchitis, emphysema, asthma, and bronchiectasis.

Chronic bronchitis, for example, involves a long-term inflammation of the bronchi in a diseased lung which may be treated by endoluminally accessing an airway and ablating the outer mucosal layer of the airway walls to destroy hyperplastic goblet cells and reduce mucus secretion. In some examples involving use of a minimally invasive ablation instrument, an objective of the treatment may be to ablate as much as the lung as possible, targeting the goblet cells at a depth of approximately 0.1-0.5 mm, without over ablating and without repeated ablation of any area. Some techniques may require multiple treatments, performed in separate procedures over days, weeks, or months.

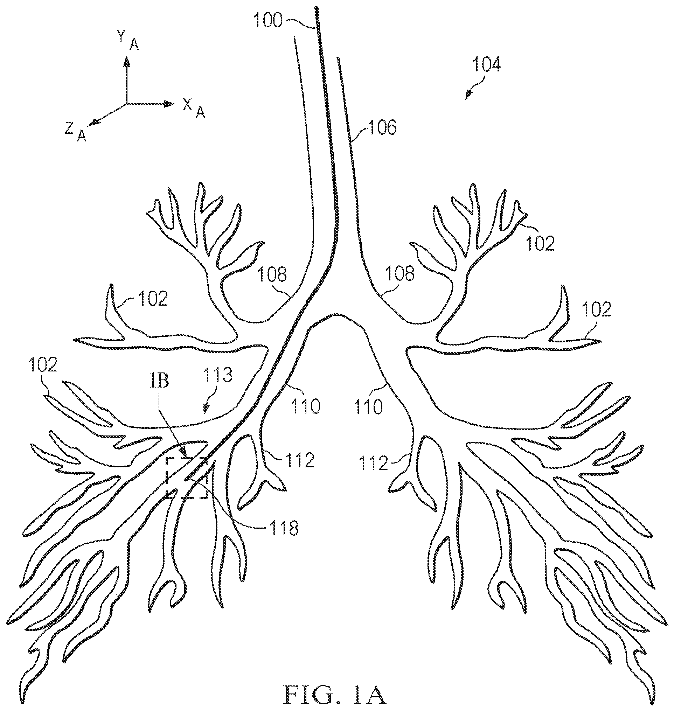

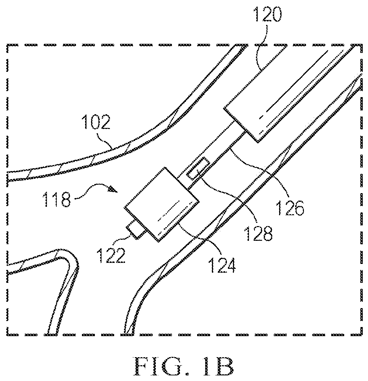

FIGS. 1A and 1B illustrate an elongated medical instrument system 100 extending within branched anatomic passageways (e.g., airways) 102 of an anatomic structure 104. In some examples the anatomic structure 104 may be a lung and the passageways 102 may include the trachea 106, primary bronchi 108, secondary bronchi 110, and tertiary bronchi 112. The anatomic structure 104 has an anatomical frame of reference (X_{A}, Y_{A}, Z_{A}). A distal end portion 118 of the medical instrument system 100 may be advanced into an anatomic opening (e.g., a patient mouth) and through the anatomic passageways 102 to perform a medical procedure, such as an endoluminal ablation treatment, at or near target tissue located in a region 113 of the anatomic structure 104. In some examples, motion and other operations of the elongated medical instrument system 100 may be controlled by a control system (e.g., control system 812) of a robot-assisted medical system (e.g., medical system 800).

In some examples, the elongated medical instrument system 100 may include a delivery catheter 120 through which extends an ablation system 122 for ablating diseased tissue. An example instrument system may be the robot-assisted bronchoscope of the Ion Platform provided by Intuitive Surgical Operations, Inc. The ablation system 122 may include an ablation device 124 located at a distal portion of an elongated shaft 126. The ablation device 124 may make contact with a wall of an anatomic passageway 102 to perform the ablation. In some examples, the ablation device 124 may be expandable, such as a balloon that is heated, fitted with one or more ablation electrodes, or configured to delivery cryo treatment. In other examples, the ablation device may include a stent-like cage, an expandable helix structure, or other structures that are heated or that includes electrodes for delivering energy to adjacent tissue. Ablation may be performed using any of a various forms of energy including radio frequency, microwave, cryo, electroporation (reversible, irreversible, thermal, or non-thermal), electrical impulses, ultrasound, direct heat, etc.

The ablation system 122 may also include a sensor system 128 for determining a position, orientation, speed, velocity, pose, shape and/or force at a distal end and/or at one or more segments along the ablation system 122. For example, the sensor system may include a position/location sensor system (e.g., an electromagnetic (EM) sensor system), a shape sensor system (e.g., an optical fiber shape sensor), and/or a force sensor system. Motion and/or operation of the delivery catheter 120, the elongated shaft 126, and/or the ablation device 124 may be controlled manually by operator manipulation and/or by a control system (e.g., control system 812). During ablation, cellular and structural changes in the epithelium and subepithelium may be induced. The ablation may cause tissue reduction, including destruction of goblet cells and cilia in lung tissue. In some examples, the cellular matrix may be preserved to allow for later regrowth of healthy cells. In some examples, the tissue reaction may occur entirely during the application of the energy, and in other examples, the tissue damage may develop over a period of time as the anatomy responds to the injury caused by the energy. In the lung, an ablation may be used to treat a variety of pulmonary conditions including lung tumors, asthma, chronic obstructive pulmonary disease, chronic bronchitis, emphysema, and bronchiectasis.

The instrument system 100 may also include a suction system including a suction lumen and a vacuum source for suctioning through the suction lumen. The suction system may be used to evacuate mucus from the patient anatomy. The instrument system 100 may also include an endoscopic imaging system, including an imaging probe, that captures images of the anatomic passageways. The instrument system 100 may be coupled to and actuated by a manipulator (e.g., the manipulator assembly 802). The instrument system or the manipulator may include actuators to control insertion, retraction and optionally, rotation or other degrees of freedom of motion of the instrument system 100 for articulation of the instrument system 100.

In some examples, the instrument system may include a robotically controlled delivery device (e.g., a robotically controlled catheter), such as the delivery catheter 120, that may deliver a passive instrument, such as a passive ablation system 122. The passive instrument may be exchanged with a suction device, or the passive instrument may include a suction port to suction mucus. The passive instrument may be exchanged for an imaging probe of the endoscopic imaging system that is used to visualize the anatomic passageway during insertion of the instrument. In other examples, the instrument (e.g., the ablation system 122) delivered by the robotically controlled delivery device (e.g., delivery catheter 120) may itself be robotically controlled and delivered through the robotically controlled delivery device. In other examples, a robotically controlled instrument (e.g., the ablation system 122) may be delivered directly without a delivery system or may be delivered through a passive sheath or other passive delivery system.

In some examples, the distal end portion 118 of the elongated medical instrument system 100 may be driven to an area of the lung starting at the most distal location within a branch to be treated, an ablation treatment may be performed, the instrument may be retracted, and an ablation treatment may be performed at the new more proximal location. In some procedures, an expandable device is used so that the device is placed at a distal location in the lungs, expanded, ablation energy is delivered, the device is collapsed, retracted to a different location and the process is repeated until as much of the lungs is ablated as desired. In other procedures, the expandable device is placed at a distal location in the lungs, expanded, ablation energy is delivered, the device is retracted during ablation energy delivery a distance to a different location, and ablation energy delivery is halted. This process can be repeated for different sections of the branch. The distal to proximal sequence may be repeated and may be performed in successive branches so that areas that have been previously ablated do not undergo further ablation. In alternative examples, depending on the disease state of the patient, similar processes can be performed proximally to distally providing for insertion of the expandable device. These techniques may be effective because missing an airway or missing an area along an airway may have minimal impact on the effectiveness of the treatment. Effectiveness of the treatment may also be dependent on the generation of airways that have been treated.

Diagnostic techniques for evaluating lung conditions may include pulmonary tests (e.g., functional tests), X-rays, and/or CT images of the anatomy. In some embodiments, computer software, alone or in combination with manual input, is used to convert the image data into a segmented two-dimensional or three-dimensional composite representation or model of a partial or an entire anatomic organ or anatomic region. The model may describe the various locations and shapes of the anatomical passages and their connectivity. More specifically, during the segmentation process the pixels or voxels may be partitioned into segments or elements or may be tagged to indicate that they share certain characteristics or computed properties such as color, density, intensity, and texture. In some embodiments, segmenting the image data may comprise selecting components to associate with certain objects or tissue types. Anatomic passageways in the lung (e.g., airways) may be segmented based on their shared characteristics. Airways that contain mucus may be classified incorrectly because of their altered characteristics and may thus be falsely interpreted as a disconnection between airways.

Ablation treatment parameters (e.g., power, duration) may be device dependent, with each treatment device having a treatment chart of recommended settings. In some examples, ablation treatment is open loop, without feedback, sensing, or other analysis to compare intended treatment with actual treatment. Rather, treatment may be delivered for a predetermined amount of time at a location then the ablation device may be moved to another location for a predetermined amount of time.

In some examples, ablation treatment may be performed by manually moving or manipulating the ablation device, but manual control may not always achieve optimal results. For example, manually dragging an ablation device along an airway wall may achieve ablation for a large surface area but dragging may be difficult to control manually. Diameters of airways may vary along the length of the airway so an expandable device may need to be altered in size, by manual manipulation, to achieve good contact as the device is retracted. It may be difficult for an operator to assess how much to expand or collapse the device in order to still achieve sufficient contact with the airway walls. In some examples, avoiding overlap of ablation lesions is preferred, but under manual operation, it may be difficult to determine where an ablation lesion has been formed. In some examples, the operator is manually moving the ablation device using only endoscopic and/or fluoroscopic imaging guidance where ablation lesions are difficult or impossible to visualize and locations of the ablation device within the lungs are difficult to comprehend. Consequently, the operator may be unsure about where to perform subsequent ablations so that sufficient tissue area is treated while not overlapping any lesions.

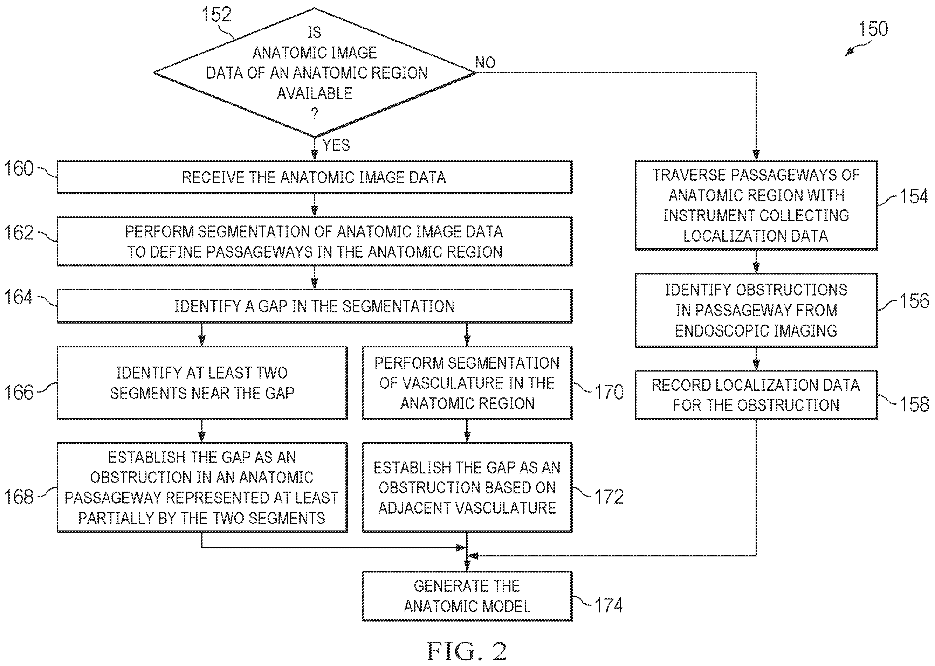

A patient treatment, such as an ablation treatment, within an anatomic passageway may be performed with an ablation system, such as instrument system 100. The treatment may be performed, for example, in a lung diseased with chronic bronchitis. FIG. 2 is a flowchart illustrating a method 150 for generating a model of an anatomic region of a patient anatomy. In some examples, the anatomic region may be the diseased lung. The method 150 and other methods described herein are illustrated as a set of operations or processes that may be performed in the same or in a different order than the order shown in the figure. Depending on the configuration of the instrument 100, one or more of the illustrated processes may be omitted in some examples of the method. Additionally, one or more processes that are not expressly illustrated in the flowcharts may be included before, after, in between, or as part of the illustrated processes. In some examples, one or more of the processes of the flowcharts may be implemented, at least in part, by a control system executing code stored on non-transitory, tangible, machine-readable media that when run by one or more processors (e.g., the processors of a control system) may cause the one or more processors to perform one or more of the processes.

The method 150 may include a process 152 of determining whether anatomic image data (e.g., X-ray and/or CT image data) of the region of the patient anatomy is available. If anatomic image data is not available, the passageways of the anatomic region may, at a process 154, be traversed with the instrument system (e.g., system 100) to collect localization data that provides survey information (e.g., airway diameter, airway length, airway orientations, and relative positions of a plurality of airways) about the configuration of the region of the patient anatomy. This survey of the region with the instrument system may be performed pre-operatively or during the treatment procedure. Further description of a surveying process is described in International Publication No. WO2021168061A1 filed February 18, 2021 (disclosing "Systems and methods for delivering targeted therapy") which is incorporated herein by reference in its entirety for all purposes. At a process 156, an obstruction in the passageway, such as a mucus plug, may be visualized in the images from the imaging system of the instrument system as the passageways are surveyed. At a process 158, localization data may be recorded by the instrument and/or the instrument control system to capture the location of the obstruction. The processes of identifying and recording the locations of obstructions may be repeated as obstructions are encountered during the traversal of the anatomic region with the instrument. At a process 174, an anatomic model may be generated from the localization data and the location of the obstructions may be marked and/or otherwise displayed within the anatomic model.

If, at process 152, anatomic image data is available, the anatomic image data may be received (for example by a control system of a robot-assisted medical system) at a process 160. At a process 162, a segmentation procedure may be performed for the image data to define passageways in the anatomic region. The segmentation procedure may identify graphical elements in the image data that represent or otherwise correspond to the anatomic structures or features. During the segmentation process, pixels or voxels generated from the image data may be partitioned into segments or elements and/or be tagged to indicate that they share certain characteristics or computed properties such as color, density, intensity, and texture. The segments or elements associated with anatomical features of the patient may be used to generate portions of the anatomic model at process 174.

At a process 164, gaps in the segmentation may be identified. To determine the nature of the gap (e.g., whether it represents a mucous plug obstruction in an airway or whether the airway is terminated), additional information from the anatomic image data may be considered. For example, at a process 166, two close segments (e.g., pixels, voxels, or contiguous groups thereof) separated by the gap may be identified. Segments may be considered close to one another based on the properties of the segments such as, similar directional vectors as defined by centerline points through the segments, a small (e.g., below a threshold) distance between centerline end points of two segments, and/or similar size (e.g., within a threshold variance) diameters for the segments. At a process 168, information about the two separated segments such as their orientation, diameter, and/or connection to other segments may be considered to establish that the gap represents an obstruction (e.g., a mucous plug, phlegm) within a continuous anatomic passageway represented by the two segments. A continuous anatomic passageway may, in some examples, be characterized by segments that have similar sized diameters, segment centerline vectors that have a similar direction, and/or segment centerline endpoints that are within a threshold distance from each other. In some examples, a gap may be determined to be an obstruction if a cylinder may be extended along a trajectory that would include both segments based on their diameters and centerlines. At the process 174, the anatomic model may be generated based on the segmentation data, and the segments separated by the gap may be connected in the anatomic model. The anatomic model may be marked to identify the location of the obstruction(s) in the anatomic model. For example, the location of mucous plugs may be identified in an anatomic model of continuous lung airways.

Additionally or alternatively, a continuous passageway (e.g., airway) that is obstructed may be determined based on the vasculature that extends along the passageway. At a process 170, segmentation of the vasculature in the anatomic region may be performed. At a process 172, the vasculature segmentation may be used to establish that the gap represents an obstruction (e.g., a mucous plug) within a continuous anatomic passageway that extends along the adjacent vasculature. At the process 174, the anatomic model may be generated based on the passageway and vasculature segmentation data, and the segments of the segmentation separated by the gap may be connected in the anatomic model with the model marked to indicate the location of the obstructions. The gap between the segments may be connected by a bridge segment to bridge the gap, and the anatomic model may include the bridge segment. The characteristics of the bridge segment may be determined from the characteristics of the adjacent segments and the gap (e.g., diameter size, length, orientation).

Optionally, if the anatomic model is used or generated during a treatment procedure, the anatomic model may be altered, improved or otherwise updated during subsequent traversal of the patient anatomy by the instrument based on new information. For example, the location of treatment delivery (e.g., the location and size of ablation lesions) may be recorded and added to the displayed model. Optionally, the location of any obstructions (e.g., mucous plugs) recorded in the anatomic model may be removed from the model if the obstructions (e.g., mucous plugs) are removed from the anatomy (e.g., by suction). In some examples, a record of the former location of obstructions may be maintained for future reference (e.g., to monitor redevelopment of obstructions in the same location). Optionally, the model may be amended to include the current location of the instrument based on operator inputs or on an instrument tracking system.

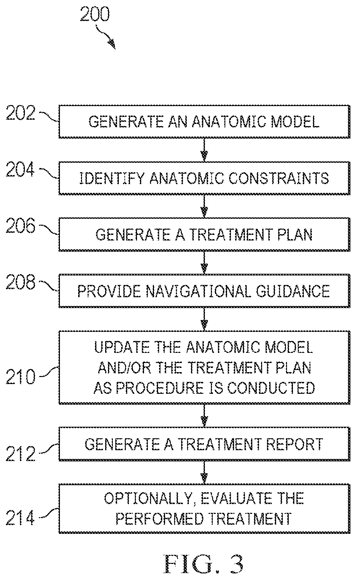

FIG. 3 is a flowchart illustrating a method 200 for performing a treatment, such as an ablation treatment. At a process 202, an anatomic model of a region of a patient anatomy may be generated or received. In some examples the anatomic model may be generated using the method 150 as depicted in the flowchart of FIG. 2. The method of treatment 200 may further include a process 204 in which anatomic constraints may be identified. The anatomic constraints may be identified from the anatomic model and may include constraints that may impede the navigation of the instrument. Anatomical constraints may include, for example, anatomic lumen diameters that are smaller than a predetermined threshold (e.g., the outer diameter of the instrument), anatomic passageways that are curved to an extent that they are difficult to navigate (e.g., a tight bend that may not be navigated based on the known flexibility or other constraints of the instrument), mucous plugs within the anatomic passageways, or sensitive anatomy such as pleura, organs, blood vessels, etc. which should be protected from ablative energy. Identifying the anatomic constraints may include visually representing the location of the anatomical constraint with a marking (e.g., a color, graphic symbol, alphanumeric text) on the displayed model.

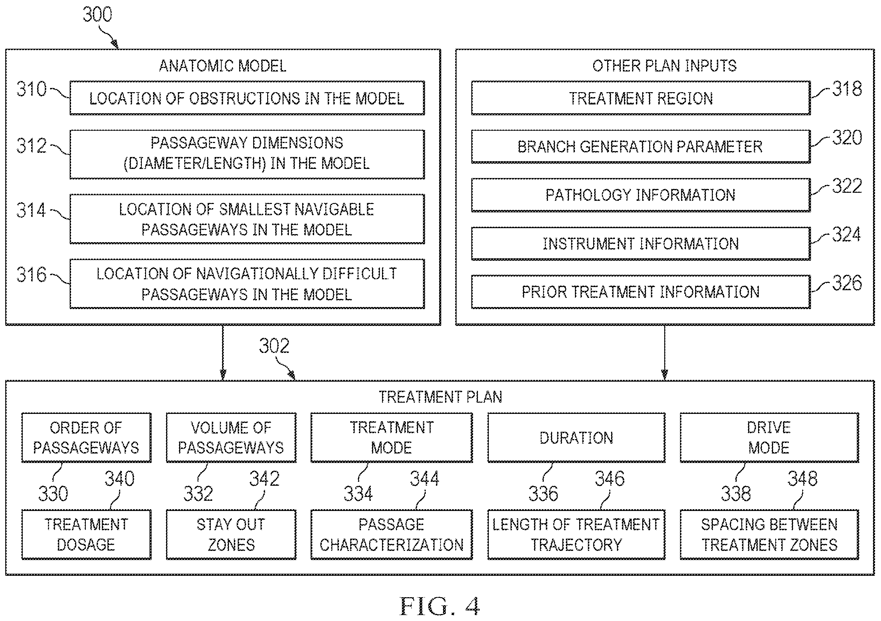

At a process 206, a treatment plan may be generated with reference to the anatomic model. FIG. 4, for example, schematically illustrates parameters from an anatomic model 300 that may be inputs to a treatment plan 302. The anatomic model 300 parameters may include anatomic constraints including the locations 310 of obstructions (e.g., mucus plugs) in the model, dimensions (e.g., diameter, length, etc.) 312 of passageways in the model, location and/or shape of smallest navigable passageways 314 in the model, and location and/or shape of navigably difficult (e.g., tortuous, narrow, sharply angled) passageways 316 in the model. Other parameters based on the anatomic model, user input, sensor inputs, or other accessible information may also or alternatively serve as inputs to the treatment plan 302. For example, a treatment area parameter 318 may be a region of the patient anatomy (e.g., a region identified on the model) identified for receiving an ablation treatment. The treatment area parameter may be determined, for example, by an operator input, prior diagnoses, or image analysis, which identify areas to be treated. In some examples, a default region may be the entire organ, such as the lung. In other examples, a default region may be a portion of the organ, such as the left lung or the right lung. Any of these parameters alone or in combination with others may serve as inputs to the treatment plan 302. A branch generation parameter 320 may identify, for example, the distal-most (e.g., farthest from the anatomic entryway) generation of passageway to be treated. The parameter 320 may be determined based, for example, on an operator input or may be based on known instrument constraints such as length of the elongated instrument, the diameter (e.g., maximum diameter or diameter at a distal portion) of the delivery or treatment instrument, and/or the minimum allowable bend radius based on the flexibility of the delivery or treatment instrument. A pathology parameter 322 may include information about the diagnosed patient condition. The information may be determined from an operator input or from an electronic medical record. The information may include a maximum allowable duration for an overall procedure time based on tolerance of the patient due to a patient condition. An instrument parameter 324 may include information about one or more delivery catheters, suction devices, treatment instruments, and/or ablation devices that may be used during a treatment. The information may include, for example, diameter and smallest bend radius. The information may include recommended dosage charts for the specific treatment or ablation instrument. A prior treatment information parameter 326 may include information from prior treatments on the same patient. For example, the prior treatment information may include locations of prior ablation treatment in the anatomic region.

A treatment plan 302 may be optimized based on the input parameters 310-326 and may include one or more optimized factors 330-348. Generally, a lung treatment plan may include delivering an ablation device to a distal portion of a target airway branch and delivering energy from the ablation device to the tissue of the airway as the ablation device is moved from the distal portion toward a defined proximal location (e.g., the location of a delivery instrument such as the delivery catheter 120). The treatment process may be repeated until all identified branches of the airways are treated. Generally, optimization of the treatment plan may prioritize treatment in areas that are more easily and quickly reached by the ablation device and/or areas that provide the greatest surface area or volume for ablation. The treatment plan may provide a predicted percentage of the anatomic region that may be treated based on the plan. The treatment plan may include a visual depiction of the overall treatment path the treatment instrument will follow. The treatment path may include a starting distal point in a first branch with a proximal retraction point for a first trajectory and a second distal point in a second branch with a second proximal retraction point for a second trajectory. Other spot treatment or trajectory starting and ending points may be provided by the treatment plan.

The factors 330-348 may be selected to optimize the treatment plan 302. A factor 330 may be an order or prioritization of anatomic branches to be treated. A factor 332 may be a volume or cumulative surface area of all the passageways to be treated. A factor 334 may be a treatment mode, such as a spot treatment mode, a defined trajectory treatment mode, or hybrid spot and trajectory treatment mode, as described in further detail below. The treatment mode may determine whether the ablation device is stationary or moving while ablation energy is delivered. A factor 336 may be a duration of the treatment or portions of the treatment. A factor 338 may be a drive mode of the treatment instrument such an automatic retraction mode, a semi-automatic retraction mode, an operator-controlled mode, a safety mode, and/or a lumen centering mode, as described in further detail below. A factor 340 may include a treatment dosage. A dosage factor may be calculated by a control system and may include power level, dwell time, and/or speed of instrument (e.g., speed of retraction along a trajectory). Determining the dosage factor may include referencing dosage charts for the ablation device. The diameter of the passageway may strongly influence the dosage. For example, a passageway with a large diameter may warrant a larger dosage than a passageway with a smaller diameter, to achieve a comparable ablation outcome. A factor 342 may include a defined stay-out zone in which treatment should be avoided. For example, areas gated by a mucus plug may be avoided or treated differently because the mucus may be difficult or time-consuming to remove or a suction or a tool change may be required. As another example, areas with passageways too small for the instrument to navigate may be in a stay-out zone. As another example, areas with a passageway shape that includes a bend that exceeds the bend radius of the instrument may be in a stay-out zone. As another example, areas with sensitive anatomy (e.g., near the pleura, organs, large blood vessels, etc.) may be stay-out zones. A factor 344 may include passage characterization. For example, the plan may identify passageways that may be characterized as relatively long, smooth, and/or straight. This characterization may provide an indication of the ease or speed with which the ablation device may be moved or dragged through the passageway. In other examples, the passageway may be characterized based on variation in the lumen diameter. This characterization may provide an indication of ease or speed with which the ablation device may traverse the passageway. For example, passageways with greater variability in diameter may require more adjustments to the ablation instrument diameter to ensure ablation contact. Such adjustments may be time-consuming or require additional calculation by an operator or a control system. A factor 346 may include the length of areas to be treated or the distance that the treatment instrument may be moved through passageway(s). A factor 348 may include spacing between treatment spots or treatment trajectories.

Referring again to FIG. 2, the method of treatment 200 may further include a process 208 in which navigational guidance may be provided during the procedure. The navigational guidance may be provided to a control system (e.g., control system 812) to activate a drive system of a robot-assisted manipulator (e.g., manipulator 802) to implement the navigational guidance. Additionally or alternatively, the navigational guidance may be provided to a user for operator-controlled navigation. The navigational guidance may include a graphical user interface that displays the anatomic model with the treatment path marked on the model. The treatment path may show locations (e.g. position points for the ablation device or a predicted contact areas on the wall of the passageway) where the instrument should be positioned for spot treatments or to begin and end trajectory treatments (e.g., in which the ablation device is moved or dragged). Trajectory treatments may be performed with an ablation device that remains actuated or under manipulator control during the motion of the ablation device to, for example, keep the ablation device centered in the lumen. Some or all of the trajectory treatment may be performed with an ablation device that is unactuated and dragged along the trajectory. In some examples, the points of travel may be displayed in numbered order to indicate the order of the branches along the treatment path. Alternatively, the display may provide only the path to the next target point.

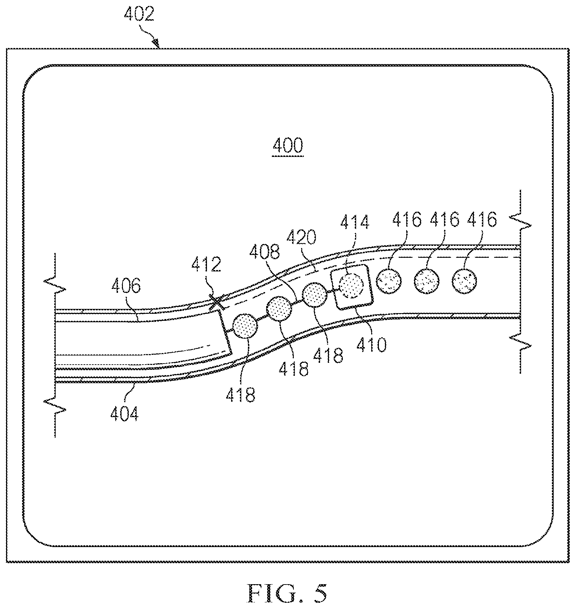

FIG. 5 illustrates a graphical user interface 400 displayed on a display system 402 (e.g., a display system 810). The graphical user interface 400 may provide navigational guidance. The graphical user interface 400 illustrates an anatomic passageway (e.g., a passageway in the anatomic model) 404 in which a delivery instrument 406 is parked. An ablation system 408 extends distally from the delivery instrument 406 and includes an ablation device 410 such as an expandable balloon, cage, or other energy delivery device. A marker 412, such as a graphic symbol or a highlighted length of the passageway, may indicate a preferred location for parking a distal end of the delivery instrument 406. A marker 414, such as a graphic symbol or a highlighted length of the passageway, may indicate the depth to which the ablation device should be extended from the delivery instrument 406 to perform an ablation treatment. Prior distal treatment locations may be designated with markers 416 to indicate the location of the prior ablations. Subsequent proximal target locations may be designated with markers 418 to indicate the location of suggested treatment locations along a treatment trajectory 420. The markers 416 may have a different color, shading, or shape from the markers 418 and the characteristic of the marker 418 may change to the characteristic of the markers 416 after treatment is performed. Alternatively, the markers 418 may be removed after the treatment occurs at the location of the markers 418. As an example, colored rings or markers may be displayed along the passageway. The marker may change color based on the location of the device in the passageway relative to the target position (e.g. green indicates an acceptable position, red indicates that the device needs to move closer to the target position). A different color may be displayed after ablation energy is delivered.

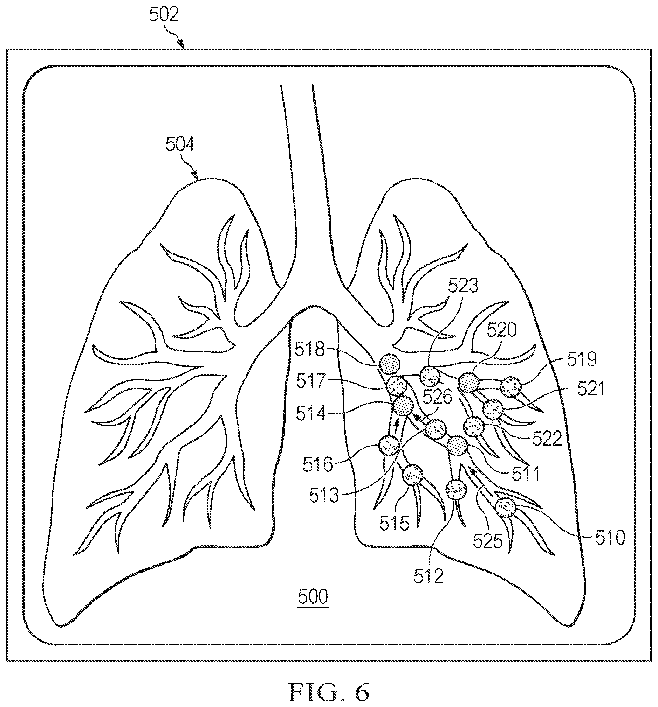

FIG. 6 illustrates a graphical user interface 500 displayed on a display system 502. The graphical user interface 500 may provide navigational guidance. The graphical user interface 500 illustrates an anatomic model 504 with markers 511, 514, 518, and 520 indicating ordered delivery instrument (e.g., instrument 120) parking locations. In some examples, the parking location for the delivery instrument may be at a carina or at the trachea. From the parking locations, an ablation device may be extended to the marked locations distal of the parking locations. For example, from the parking location 511, the ablation device (e.g., ablation device 124) may be first extended to the marker 510 for treatment. The ablation device may be operated under controlled motion (e.g., actuated movement of the ablation device) or may be dragged (e.g., passive movement) along the trajectory 525 toward the parking location 511. The movements may be, for example, manually actuated, robotically-assisted in response to operator control, or robotically-guided based on a programmed trajectory. From the parking location 511, the ablation device may next be extended to the location 512 for a spot (e.g., a discrete-location) ablation treatment. Next, the delivery device may be moved proximally to the parking location 514. The ablation device may be extended from the location 514 to the location 513 and may move along a trajectory 526 back to the parking location 514. The ablation device may then be extended from location 514 to perform a spot treatment at location 515. From a location 516, the ablation device may be operated or dragged along a trajectory toward the parking location 514. Next, the delivery device may be moved proximally to a parking location 518. From the parking location 518, the ablation device may be extended to perform a spot treatment at a location 517. Next, the delivery device may be moved distally to a parking location 520 and the ablation device may be extended to perform spot treatments at locations 519 and 521. The delivery device may then be withdrawn proximally to the location 518, and the ablation device may be extended to perform spot treatments at locations 522 and 523. After treatment, the marker at the treatment location may change color, shading, or shape or may be removed entirely from the display. In some examples, only the marker for the next location of the ablation device may be displayed. In some examples, the ablation device may be energized and retracted along a length of an airway, for example from location 510 to just distal of location 511, or from location 515 to location 516, such that a length of the airway is treated in a single energy delivery event.

Referring again to FIG. 2, the method of treatment 200 may further include a process 210 in which the anatomic model or the treatment plan may be updated as the treatment procedure is conducted. The ablation device may be tracked to provide real-time localization of the device and the localization information may be registered to and, optionally, displayed on the anatomic model. Indicia of efficacy may be displayed with reference to the anatomic model. For example, efficacy may be determined based on measures of friction or pull force, applied power, duration of energy delivery, and/or the specifications of the ablation device. The treatment plan may be updated. For example, treatment locations in the pre-procedure treatment plan (e.g., plan 302) may be compared to the actual treatment location in the implemented plan. The treatment plan may be updated during the procedure based on the actual treatment location so that treatment areas do not overlap. As the procedure is conducted, an indicator (e.g., audible, haptic, visual) may be presented if an area that has been previously treated is about to undergo additional or overlapping treatment.

At a process 212, a treatment report may be generated. In some examples, the treatment report may be generated after the ablation treatment has been completed. The treatment report may include an image of the anatomic model (full anatomic model or portion thereof) with the treated areas marked including duration and power levels of treatment and/or an evaluation of ablation efficacy correlated with specific locations within the model. The treatment report may provide a measurement (e.g., a percentage) of the passageways in the anatomic region that were treated compared to the expected measurement of the passageways to be treated. The measure of actual treatment area compared to the predicted treatment area may be used to evaluate the performed treatment.

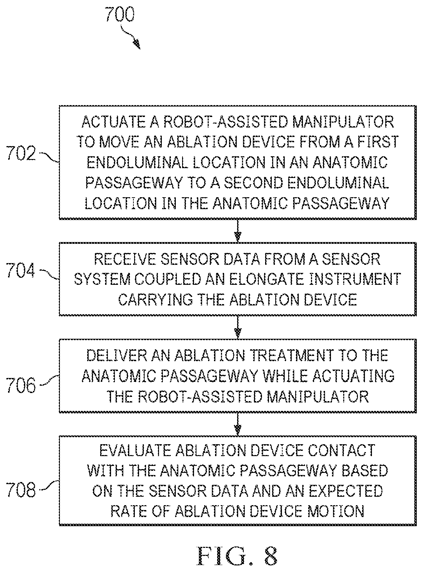

Optionally, an updated model from the treatment report may be used to plan future treatment procedures on the same patient and/or to determine areas that were omitted in the original procedure that should be treated in subsequent treatment procedures. Optionally, anew anatomic model at a future point in time may be created and may be overlaid or otherwise compared to the updated model from the treatment report to develop a new treatment plan for a subsequent treatment procedure. During the subsequent procedure, indicators (e.g., audible, haptic, visual) may be presented to avoid treatment in previously treated areas. Optionally, at a process 214 the efficacy of the treatment may be evaluated. An example of a method for evaluating efficacy is provided at FIG. 8, as will be described in detail below.

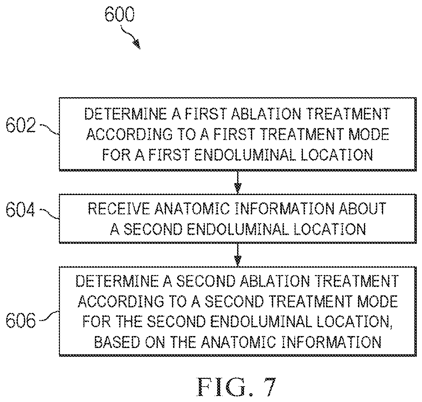

FIG. 7 provides a flowchart illustrating a method 600 for determining ablation treatment using first and second treatment modes. Examples of treatment modes include a spot treatment mode, a trajectory treatment mode, or a hybrid spot and trajectory treatment mode. The determined treatment modes (as described below) may be used to generate and/or optimize the treatment plan (e.g., via treatment mode factor 334) and provide navigational guidance as described in processes 206 and 208 above. At a process 602, a first ablation treatment according to a first treatment mode may be determined for an ablation device. The first ablation treatment may be performed at a first endoluminal treatment location (e.g., a location at marker 414 in Fig. 5). At a process 604, anatomic information may be received about a second endoluminal location. The anatomic information may include the sensitivity of the anatomy, lumen diameter, tightness of the lumen bends, or other information about the ease, difficulty, or risk of harm of movement through the passageway. The anatomic information may be determined from the anatomic model 300 inputs 310-316 or other inputs 318-326. The second endoluminal location may be a location for a spot ablation or may be a location along a trajectory of movement of the ablation device. At a process 606, a second treatment according to a second treatment mode may be determined for the ablation device. The second ablation treatment may be performed at the second endoluminal location (e.g., a location at a marker 418 in Fig. 5), based on the anatomic information. For example, if the anatomic information indicates that the anatomy includes sensitive areas, an irregular diameter, or tight bends that exceed a bending threshold, a spot treatment mode may be determined. Alternatively, a defined trajectory treatment mode may be used if the anatomic information indicates that the anatomy between the first and second endoluminal locations includes a length of passageway that is relatively straight (e.g., below the bending threshold) and/or has a uniform diameter. The processes 604 and 606 may be repeated multiple times during an ablation procedure.

Treatment modes may include a spot treatment mode in which an ablation device is positioned at first target and the ablation device engages the wall of the anatomic lumen (e.g., device is expanded if a balloon, cage, or expandable electrode). Ablation energy is delivered while the ablation device engages the wall. After the spot treatment, the ablation device disengages the wall of the anatomic lumen (e.g., the device is collapsed if expandable). The ablation device is retracted to the next target point and the process is repeated. In some examples, targets may be spaced a predefined distance apart for each treatment. In some examples, targets may be varied depending on the parameters of the ablation device and the response of the ablation device to the characteristics of the lumen at a location. Examples of such characteristics may include a diameter of the lumen, level of moisture, flexibility or expandability of the lumen in that location, tissue characteristics, and/or tissue depth. The targets may be user defined or may be determined based on a maximum allowed distance for the desired outcome.

Treatment modes may also include a defined trajectory treatment mode in which the ablation device is positioned at first distal target and the ablation device engages the wall of the anatomic lumen (e.g., device is expanded if a balloon, cage, or expandable electrode). Ablation energy may be delivered while the device is simultaneously retracted until the device has reached the next target point. The movement of the ablation device may be controlled such that the manipulator assembly may make adjustments to orientation, dimensions, or other properties of the active ablation device as the device is retracted. The adjustments may be chosen to maintain the ablation device along a lumen centerline. Alternatively, the ablation device may be passively dragged along the anatomic lumen. In some examples, the deployment of the ablation device may be optimized by maintaining the shaft in a consistent orientation with respect to the anatomic lumen. The next target may be the nearest proximal carina, the trachea, and/or the parking location of the delivery device. In some examples, the next target could be a predetermined distance proximal to the previous target where the system pauses before another treatment.

Treatment modes may also include a hybrid treatment mode in which the treatment modes are switched depending on the anatomy. For example, in a defined trajectory treatment mode it may be difficult to drag the ablation device around tight bends, so the mode may be switched to a spot mode. As another example, sensitive anatomy may be present so spot ablations could be provided around the sensitive anatomy. As another example, spot ablations may be used in small airways to avoid over-ablation that may occur using the defined trajectory treatment mode. In a further example, where diameter size of lumens changes drastically spot ablations may be used to avoid the need to continually change diameter size of the expandable element during a trajectory treatment.

Dosage (e.g., a dosage factor 340) may vary during the treatment modes depending on the anatomy. For example, device expansion, treatment duration and dwell time, power, and retraction speed may vary based on passageway size. In some examples, the diameter of passageways may be determined during the procedure by imaging systems such as endoscopic camera imaging, ultrasound imaging, intra-operative CT, or optical coherence tomography (OCT). In some examples, airway size may be based on a registered map. For example, the anatomic model (e.g., the model generated at process 152) may serve as a map which is registered to the instrument. Based on the location of the instrument within the anatomic model, the diameter of the airway may be calculated. Airway size may also be based on sensors of the sensor system detecting contact with airway wall (e.g., pressure sensors, force sensors, impedance sensors, contact sensors, and/or an optical fiber integrated into external layer of an expandable ablation device). In some examples, ablation instruments may include dosage charts that provide recommended dosages.

Drive modes (e.g., a drive mode factor 338) for controlling the motion of the ablation device may also vary during the planned ablation procedure. In an automatic retraction drive mode, an operator may position an ablation device at first target and provide an input to start treatment. The full treatment, including all ablation device movement or adjustment may be performed automatically by a control system. In a semi-automatic drive move, an operator may position the device at a first target and provide an input (e.g., a button press). The control system may perform treatment as long as the input is triggered but may stop as soon as input is released or final target is reached. In an operator-controlled drive mode, a graphical user interface may provide an indicator of when to treat, when to retract, and when to stop treatment. For example, an operator may engage separate "treat", "stop treatment", or "retract" buttons. Activation of the "treat" button may expand the ablation device and cause the ablation device to deliver energy. Activation of the "stop treatment" button may stop energy delivery, and in some examples may collapse the ablation device. Alternatively, a first push activation of the "treat" button may begin treatment, and a second push activation may end treatment. Activation of the "retract" button may cause the ablation device to move from a distal point to a more proximal point. In some examples, operations may be performed only when the button is depressed. In some embodiments, the system may include an insertion drive mode, and an "insert" button may be provided. Activation of the "insert" button may cause insertion of the device such that treatment is provided from a proximal to distal location in anatomy.

In any drive mode, treatment, retraction, and/or insertion may be stopped if a disturbance condition is met (e.g., a FRL, error position, force above a threshold, or a visual cue). In any drive mode, retraction may include automatically centering the catheter/instrument in the middle of the airway. The centering may be based on an endoluminal camera view. Analysis of the video image may be used to detect hole structures and to detect whether the catheter is pointing towards the centers of the hole structures. An operator may look at a real time view and adjust articulation of the ablation device. In some examples, the centering may be based on closing a positional loop on the position within a registered model with the measured position of the catheter using, for example, one or more location sensors. In some examples, the centering may be based on detected position of the instrument relative to the airway wall (e.g., using pressure sensors, force sensors, EM sensors, impedance sensors, contact sensors, and/or an optical fiber integrated into external layer of an expandable ablation device).

FIG. 8 provides a flowchart illustrating a method 700 for evaluating the efficacy of a treatment. At a process 702, a robot-assisted manipulator (e.g., the manipulator system 802) may be actuated to move an ablation device from a first endoluminal location in an anatomic passageway to a second endoluminal location in the anatomic passageway. At a process 704, sensor data may be received from a sensor system coupled to an elongate instrument carrying the ablation device. At a process 706, an ablation treatment may be delivered to the anatomic passageway while actuating the robot-assisted manipulator. At a process 708, contact between the ablation device and the anatomic passageway may be evaluated based on the sensor data and an expected rate of ablation device motion. The process 708 may be used, for example, to evaluate the efficacy of the process 164 of method 150.

The sensor data may include localization sensor data that may be used to help evaluate ablation efficacy. Sensor data may include, for example, shape information for the elongate instrument, location/position information for the ablation device, velocity information for the ablation device (which may include direction), actuator data, and/or force information for the ablation device. For example, a measure of friction during dragging or other motion along a defined trajectory may be determined by measuring retraction actuator data and localization data. If the actuator data indicates the ablation device is moving in a retraction direction through a passageway, but a shape sensor or position sensor indicates that no movement has occurred, over-treatment maybe be happening or may have happened in that area of the passageway. If the actuator data indicates the ablation device is moving in a retraction direction through a passageway and there is a sudden movement of distal tip of catheter, then the length of the passageway that was passed rapidly by the ablation device may be under-treated. Contact with the wall of the airway may be determined by measuring the actuator effort during retraction and then subtracting the contributions of catheter friction and catheter bending to determine the pulling force. A relatively high pulling force or a pulling force exceeding a pull force threshold value may indicate a good or acceptable contact which may correlate with a good ablation. A relatively low pulling force or a pulling force below a pull force threshold value may indicate poor, slight, or unacceptable contact which may correlate with a poor ablation. In some examples, measurements of location compared to time may be used instead of retraction actuator data. The pull force may be identified with distal sensing on the ablation instrument.

In some examples, the ablation treatment may be performed without an anatomic model or a treatment plan, but the ablation treatment locations may be tracked and localized using location sensors coupled to the treatment device (or instrument) providing data in a localization sensor frame of reference. In one example, the user can be provided an alert (audible, visual, and/or haptic) which indicates that an area has been previously treated to avoid repeated or overlapped treatments. A record of the ablation treatment locations may serve as inputs to a subsequent anatomic model and/or treatment plan. In some embodiments, if the treatment device is registered to the patient anatomy during an initial procedure (e.g., registered to known anatomical landmarks in a patient anatomy such as the main carina and main bifurcations) and then re-registered to the patient anatomy in a follow up procedure, the record of ablation treatment locations can be used during the follow up procedure. During the follow up procedure, alerts can be provided (e.g., visual, audible, or haptic) which indicate that a location was previously treated.

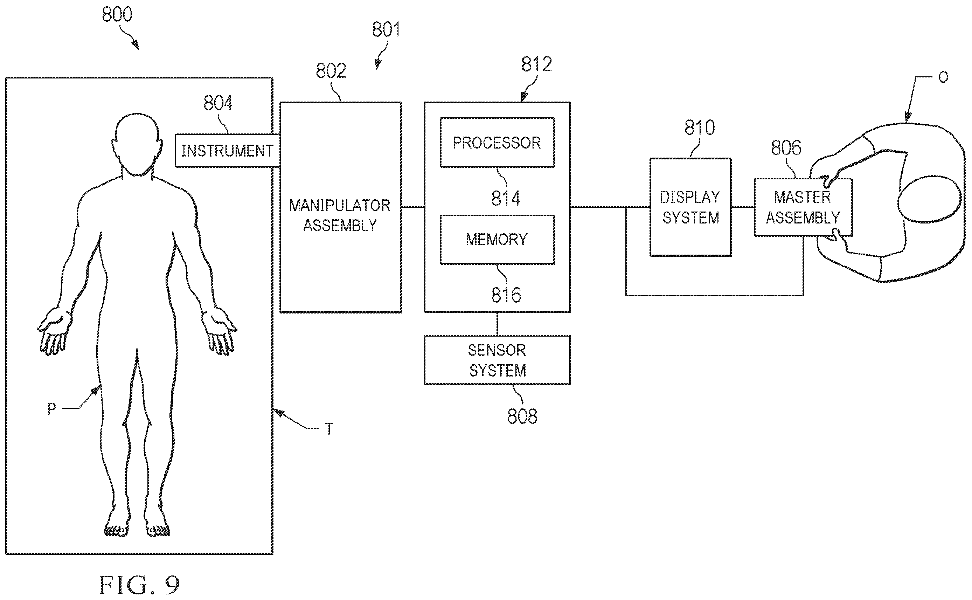

In some examples, the systems and methods disclosed herein may be used in a medical procedure performed with a robot-assisted medical system as described in further detail below. As shown in FIG. 9, a robot-assisted medical system 800 may include a manipulator assembly 802 for operating a medical instrument 804 (e.g., medical instrument system 100, the delivery instrument, treatment device, or any of the instruments described herein) in performing various procedures on a patient P positioned on a table T in a surgical environment 801. The manipulator assembly 802 may be teleoperated, non-teleoperated, or a hybrid teleoperated and non-teleoperated assembly with select degrees of freedom of motion that may be motorized and/or teleoperated and select degrees of freedom of motion that may be non-motorized and/or non-teleoperated. A master assembly 806, which may be inside or outside of the surgical environment 801, generally includes one or more control devices for controlling manipulator assembly 802. Manipulator assembly 802 supports medical instrument 804 and may optionally include a plurality of actuators or motors that drive inputs on medical instrument 804 in response to commands from a control system 812. The actuators may optionally include drive systems that when coupled to medical instrument 804 may advance medical instrument 804 into a naturally or surgically created anatomic orifice. Other drive systems may move the distal end of medical instrument in multiple degrees of freedom, which may include three degrees of linear motion (e.g., linear motion along the X, Y, Z Cartesian axes) and in three degrees of rotational motion (e.g., rotation about the X, Y, Z Cartesian axes). The manipulator assembly 802 may support various other systems for irrigation, treatment, or other purposes. Such systems may include fluid systems (including, for example, reservoirs, heating/cooling elements, pumps, and valves), generators, lasers, interrogators, and ablation components.

Robot-assisted medical system 800 also includes a display system 810 for displaying an image or representation of the surgical site and medical instrument 804 generated by a sensor system 808 which may include an endoscopic imaging system. Display system 810 and master assembly 806 may be oriented so an operator O can control medical instrument 804 and master assembly 806 with the perception of telepresence. Any of the previously described graphical user interfaces may be displayable on a display system 810 and/or a display system of an independent planning workstation.

The sensor system 808 may include a position/location sensor system (e.g., an actuator encoder or an electromagnetic (EM) sensor system) and/or a shape sensor system (e.g., an optical fiber shape sensor) for determining the position, orientation, speed, velocity, pose, and/or shape of the medical instrument 804. The sensor system 808 may also include temperature, pressure, force, or contact sensors or the like. Data from the sensor system 808 may be used to generate survey information about the configuration of the region of the patient anatomy, including airway diameter, airway length, airway orientations, and relative positions of a plurality of airways.

Robot-assisted medical system 800 may also include control system 812. Control system 812 includes at least one memory 816 and at least one computer processor 814 for effecting control between medical instrument 804, master assembly 806, sensor system 808, and display system 810. Control system 812 also includes programmed instructions (e.g., a non-transitory machine-readable medium storing the instructions) to implement a plurality of operating modes of the robot-assisted medical system including a navigation planning mode, a navigation mode, and/or a procedure mode. Control system 812 also includes programmed instructions (e.g., a non-transitory machine-readable medium storing the instructions) to implement some or all of the processes described in accordance with aspects disclosed herein, including, for example, expanding the expandable device, regulating the temperature of the heating system, regulating valves to control fluid delivery, controlling fluid flow rate, controlling insertion and retraction of the treatment instrument, controlling actuation of a distal end of the treatment instrument, receiving sensor information, altering signals based on the sensor information, selecting a treatment location, and/or determining a size to which the expandable device may be expanded.

Control system 812 may optionally further include a virtual visualization system to provide navigation assistance to operator O when controlling medical instrument 804 during an image-guided surgical procedure. Virtual navigation using the virtual visualization system may be based upon reference to an acquired pre-operative or intra-operative dataset of anatomic passageways. The virtual visualization system processes images of the surgical site imaged using imaging technology such as computerized tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. The control system 812 may use a pre-operative image to locate the target tissue (using vision imaging techniques and/or by receiving user input) and create a pre-operative plan, including an optimal first location for performing bronchial passageway and vasculature occlusion. The pre-operative plan may include, for example, a planned size to expand the expandable device, a treatment duration, a treatment temperature, and/or multiple deployment locations.

In the description, specific details have been set forth describing some examples. Numerous specific details are set forth to provide a thorough understanding of the examples. It will be apparent, however, to one skilled in the art that some examples may be practiced without some or all these specific details. The specific examples disclosed herein are meant to be illustrative but not limiting. One skilled in the art may realize other elements that, although not specifically described here, are within the scope and the spirit of this disclosure.

Elements described in detail with reference to one example, implementation, or application optionally may be included, whenever practical, in other examples, implementations, or applications in which they are not specifically shown or described. For example, if an element is described in detail with reference to one example and is not described with reference to a second example, the element may nevertheless be claimed as included in the second example. Thus, to avoid unnecessary repetition in the following description, one or more elements shown and described in association with one example, implementation, or application may be incorporated into other examples, implementations, or aspects unless specifically described otherwise, unless the one or more elements would make an example or implementation non-functional, or unless two or more of the elements provide conflicting functions. Not all the illustrated processes may be performed in all examples of the disclosed methods. Additionally, one or more processes that are not expressly illustrated in may be included before, after, in between, or as part of the illustrated processes. In some examples, one or more of the processes may be performed by a control system or may be implemented, at least in part, in the form of executable code stored on non-transitory, tangible, machine-readable media that when run by one or more processors may cause the one or more processors to perform one or more of the processes.

Any alterations and further modifications to the described devices, instruments, methods, and any further application of the principles of the present disclosure are fully contemplated as would normally occur to one skilled in the art to which the disclosure relates. In addition, dimensions provided herein are for specific examples and it is contemplated that different sizes, dimensions, and/or ratios may be utilized to implement the concepts of the present disclosure. To avoid needless descriptive repetition, one or more components or actions described in accordance with one illustrative example can be used or omitted as applicable from other illustrative examples. For the sake of brevity, the numerous iterations of these combinations will not be described separately. For simplicity, in some instances the same reference numbers are used throughout the drawings to refer to the same or like parts.

The systems and methods described herein may be suited for imaging, via natural or surgically created connected passageways, in any of a variety of anatomic systems, including the lung, colon, the intestines, the stomach, the liver, the kidneys and kidney calices, the brain, the heart, the circulatory system including vasculature, and/or the like. While some examples are provided herein with respect to medical procedures, any reference to medical or surgical instruments and medical or surgical methods is non-limiting. For example, the instruments, systems, and methods described herein may be used for non-medical purposes including industrial uses, general robotic uses, and sensing or manipulating non-tissue work pieces. Other example applications involve cosmetic improvements, imaging of human or animal anatomy, gathering data from human or animal anatomy, and training medical or non-medical personnel. Additional example applications include use for procedures on tissue removed from human or animal anatomies (without return to a human or animal anatomy) and performing procedures on human or animal cadavers. Further, these techniques can also be used for surgical and nonsurgical medical treatment or diagnosis procedures.

One or more elements in examples of this disclosure may be implemented in software to execute on a processor of a computer system such as control processing system. When implemented in software, the elements of the examples of this disclosure may be code segments to perform various tasks. The program or code segments can be stored in a processor readable storage medium or device that may have been downloaded by way of a computer data signal embodied in a carrier wave over a transmission medium or a communication link. The processor readable storage device may include any medium that can store information including an optical medium, semiconductor medium, and/or magnetic medium. Processor readable storage device examples include an electronic circuit; a semiconductor device, a semiconductor memory device, a read only memory (ROM), a flash memory, an erasable programmable read only memory (EPROM); a floppy diskette, a CD-ROM, an optical disk, a hard disk, or other storage device. The code segments may be downloaded via computer networks such as the Internet, Intranet, etc. Any of a wide variety of centralized or distributed data processing architectures may be employed. Programmed instructions may be implemented as a number of separate programs or subroutines, or they may be integrated into a number of other aspects of the systems described herein. In some examples, the control system may support wireless communication protocols such as Bluetooth, Infrared Data Association (IrDA), HomeRF, IEEE 802.11, Digital Enhanced Cordless Telecommunications (DECT), ultra-wideband (UWB), ZigBee, and Wireless Telemetry.

Note that the processes and displays presented might not inherently be related to any particular computer or other apparatus. Various general-purpose systems may be used with programs in accordance with the teachings herein, or it may prove convenient to construct a more specialized apparatus to perform the operations described. The required structure for a variety of these systems will appear as elements in the claims. In addition, the examples of the invention are not described with reference to any particular programming language. It will be appreciated that a variety of programming languages may be used to implement the teachings of the invention as described herein.

This disclosure describes various instruments, portions of instruments, and anatomic structures in terms of their state in three-dimensional space. As used herein, the term position refers to the location of an object or a portion of an object in a three-dimensional space (e.g., three degrees of translational freedom along Cartesian x-, y-, and z-coordinates). As used herein, the term orientation refers to the rotational placement of an object or a portion of an object (e.g., in one or more degrees of rotational freedom such as roll, pitch, and/or yaw). As used herein, the term pose refers to the position of an object or a portion of an object in at least one degree of translational freedom and to the orientation of that object or portion of the object in at least one degree of rotational freedom (e.g., up to six total degrees of freedom). As used herein, the term shape refers to a set of poses, positions, or orientations measured along an object.

While certain illustrative examples of the invention have been described and shown in the accompanying drawings, it is to be understood that such examples are merely illustrative of and not restrictive on the broad invention, and that the examples of the invention are not limited to the specific constructions and arrangements shown and described, since various other modifications may occur to those ordinarily skilled in the art.

Various aspects of the subject matter described herein are set forth in the following numbered examples.

Example 1. A system comprises a robot-assisted manipulator, an ablation device configured for coupling to the robotic-assisted manipulator, and a control system coupled to the robot-assisted manipulator. The control system is configured to determine a first ablation treatment according to a first treatment mode for a first endoluminal location, receive anatomic information about a second endoluminal location, and based on the anatomic information, determine a second ablation treatment according to a second treatment mode for the second endoluminal location.

Example 2. The system of example 1, wherein the anatomic information includes an evaluation of diameter uniformity between the first endoluminal location and the second endoluminal location.

Example 3. The system of example 2, wherein the second ablation treatment is determined to be a spot treatment mode if the diameter uniformity is irregular.

Example 4. The system of example 2, wherein the second ablation treatment is determined to be a defined trajectory treatment mode if the diameter uniformity is consistent.

Example 5. The system of example 1, wherein the anatomic information includes an evaluation of passageway bending between the first endoluminal location and the second endoluminal location.

Example 6. The system of example 5, wherein the second ablation treatment is determined to be a spot treatment mode if the passageway bending exceeds a bending threshold.

Example 7. The system of example 5, wherein the second ablation treatment is determined to be a defined trajectory treatment mode if the passageway bending is below a bending threshold.

Example 8. The system of example 1, wherein the first or second treatment mode is a spot treatment mode in which the ablation device is stationary during an application of energy.

Example 9. The system of example 1, wherein the first or second treatment mode is a defined trajectory treatment mode in which the ablation device moves along a defined trajectory during an application of energy.

Example 10. A system comprises a robot-assisted manipulator, an elongate instrument configured for manipulation by the robot-assisted manipulator, an ablation device coupled to a distal portion of the elongate instrument, a sensor system coupled to the elongate instrument, and a control system coupled to the robot-assisted manipulator. The control system is configured to actuate the robot-assisted manipulator to move the ablation device from a first endoluminal location in an anatomic passageway to a second endoluminal location in the anatomic passageway, wherein an expected rate of ablation device motion is associated with the actuation of the robot-assisted manipulator. The control system is also configured to receive sensor data from the sensor system while actuating the robot-assisted manipulator, deliver an ablation treatment to the anatomic passageway while actuating the robot-assisted manipulator, and based on the received sensor data and the expected rate of ablation device motion, evaluate ablation device contact with the anatomic passageway.

Example 11. The system of example 10, wherein the sensor data includes shape information for the elongate instrument.

Example 12. The system of example 10 or example 11, wherein the sensor data includes location information for the ablation device.

Example 13. The system of any one of examples 10-12, wherein the sensor data includes velocity information for the ablation device.

Example 14. The system of any one of examples 10-13, wherein the sensor data includes force information for the ablation device.

Example 15. The system of example 14, wherein the force information includes a measure of pull force and wherein ablation device contact is evaluated as acceptable if the pull force exceeds a pull force threshold.

Example 16. The system of example 14, wherein the force information includes a measure of pull force and wherein ablation device contact is evaluated as unacceptable if the pull force is below a pull force threshold.

Example 17. The system of any one of examples 10-16, wherein the evaluation includes determining from the sensor data that an actual ablation device motion was less than the expected rate of ablation device motion and the ablation device contact was longer than expected.

Example 18. The system of any one of examples 10-16, wherein the evaluation includes determining from the sensor data that an actual ablation device motion was greater than the expected rate of ablation device motion and the ablation device contact was shorter than expected.

Example 19. The system of example 18 wherein the control system is further configured to deliver another ablation treatment to the anatomic passageway when the evaluation determines that the ablation device contact was shorter than expected.

Example 20. The system of any one of examples 10-19, further comprising: a delivery catheter coupled to the robot-assisted manipulator, wherein the elongate instrument extends through the delivery catheter.

### PREFERRED EMBODIMENTS

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A system comprising:
   a processor; and
   a memory having computer readable instructions stored thereon, the computer readable instructions, when executed by the processor, cause the system to:
      receive anatomic image data for an anatomic area;
      segment the anatomic image data to identify an anatomic passageway in the anatomic area;
      identify a gap between a first segment and a second segment of the segmented anatomic image data;
      generate a bridge segment to bridge the gap; and
      generate an anatomic model of a diseased lung including the first segment, the second segment and the bridge segment.
2. The system of clause 1, wherein the computer readable instructions, when executed by the processor, further cause the system to determine a characteristic of the bridge segment based on one or more of the first segment, the second segment, or the gap.
3. The system of clause 2, wherein the characteristic of the bridge segment comprises a bridge diameter determined from the first segment and the second segment.
4. The system of clause 2 or clause 3, wherein the characteristic of the bridge segment comprises a bridge length determined from a length of the gap.
5. The system of any one of clauses 1-4, wherein the computer readable instructions, when executed by the processor, further cause the system to segment vasculature in the anatomic area, and wherein identifying the gap between the first segment and the second segment includes determining that the segmented vasculature extends between the first segment and the second segment.
6. The system of any one of clauses 1-5, wherein the gap is associated with a mucus plug in the anatomic area.
7. A system comprising:
   a processor; and
   a memory having computer readable instructions stored thereon, the computer readable instructions, when executed by the processor, cause the system to:
      identify a plurality of passageways in an anatomic region;
      identify at least one area of anatomic constraint in the plurality of passageways; and
      determine an ablation treatment plan based on the at least one area of anatomic constraint, the ablation treatment plan including a treatment order for the passageways and a plurality of sequential treatment locations.
8. The system of clause 7, wherein the plurality of sequential treatment locations includes a start treatment location at which a first ablation treatment of the ablation treatment plan is delivered and a stop treatment location at which a final ablation treatment of the ablation treatment plan is delivered.
9. The system of clause 7 or clause 8, wherein the area of anatomic constraint includes an obstruction in a passageway of the plurality of passageways.
10. The system of any one of clauses 7-9, wherein the area of anatomic constraint includes a passageway diameter size for a passageway of the plurality of passageways that is smaller than a diameter of an instrument used in the ablation treatment plan.
11. The system of any one of clauses 7-10, wherein the area of anatomic constraint includes a shape of a passageway of the plurality of passageways that exceeds a bend radius of an instrument used in the ablation treatment plan.
12. The system of any one of clauses 7-11, wherein the ablation treatment plan includes one or more treatment modes.
13. The system of any one of clauses 7-12, wherein the ablation treatment plan includes a duration of treatment.
14. The system of any one of clauses 7-13, wherein the ablation treatment plan includes a drive mode for an instrument used in the ablation treatment plan.
15. The system of any one of clauses 7-14, wherein the ablation treatment plan includes a treatment dosage.
16. The system of any one of clauses 7-15, wherein the ablation treatment plan includes a stay-out zone through which an instrument used in the ablation treatment plan is not to traverse.
17. The system of any one of clauses 7-16, wherein the ablation treatment plan includes a distance that an instrument used in the ablation treatment plan is moved.
18. The system of any one of clauses 7-17, wherein the ablation treatment plan includes a spacing distance between the sequential treatment locations.
19. A system comprising:
   a processor; and
   a memory having computer readable instructions stored thereon, the computer readable instructions, when executed by the processor, cause the system to:
      generate an ablation treatment plan;
      provide navigation guidance to perform an ablation treatment according to the ablation treatment plan; and
      generate a treatment report after performance of the ablation treatment according to the ablation treatment plan.
20. The system of clause 19, wherein the treatment report includes an image of an anatomic model with a treatment marker indicating an ablation location generated during the ablation treatment.
21. The system of clause 20, wherein the treatment marker includes a duration or a power level of the ablation treatment at the ablation location.
22. The system of clause 20 or clause 21, wherein the computer readable instructions, when executed by the processor, cause the system to:
   generate a second ablation treatment plan based on the image of the anatomic model with the treatment marker.
23. The system of any one of clauses 19-22, wherein the treatment report includes a measure of an actual treatment area for the performed ablation treatment compared to a predicted treatment area from the ablation treatment plan.

## Claims

1. A system comprising:
a processor; and
a memory having computer readable instructions stored thereon, the computer readable instructions, when executed by the processor, cause the system to:
identify a plurality of passageways in an anatomic region;
identify at least one area of anatomic constraint in the plurality of passageways;
and
determine an ablation treatment plan based on the at least one area of anatomic constraint, the ablation treatment plan including a treatment order for the passageways and a plurality of sequential treatment locations.

2. The system of claim 1, wherein the plurality of sequential treatment locations includes a start treatment location at which a first ablation treatment of the ablation treatment plan is delivered and a stop treatment location at which a final ablation treatment of the ablation treatment plan is delivered.

3. The system of claim 1 or claim 2, wherein the area of anatomic constraint includes an obstruction in a passageway of the plurality of passageways.

4. The system of any one of claims 1-3, wherein the area of anatomic constraint includes a passageway diameter size for a passageway of the plurality of passageways that is smaller than a diameter of an instrument used in the ablation treatment plan.

5. The system of any one of claims 1-4, wherein the area of anatomic constraint includes a shape of a passageway of the plurality of passageways that exceeds a bend radius of an instrument used in the ablation treatment plan.

6. The system of any one of claims 1-5, wherein the ablation treatment plan includes one or more treatment modes.

7. The system of any one of claims 1-6, wherein the ablation treatment plan includes a duration of treatment.

8. The system of any one of claims 1-7, wherein the ablation treatment plan includes a drive mode for an instrument used in the ablation treatment plan.

9. The system of any one of claims 1-8, wherein the ablation treatment plan includes a treatment dosage.

10. The system of any one of claims 1-9, wherein the ablation treatment plan includes a stay-out zone through which an instrument used in the ablation treatment plan is not to traverse.

11. The system of any one of claims 1-10, wherein the ablation treatment plan includes a distance that an instrument used in the ablation treatment plan is moved.

12. The system of any one of claims 1-11, wherein the ablation treatment plan includes a spacing distance between the sequential treatment locations.

13. A non-transitory machine-readable media storing instructions that, when executed by one or more processors, cause the one or more processors to:
identify a plurality of passageways in an anatomic region;
identify at least one area of anatomic constraint in the plurality of passageways; and
determine an ablation treatment plan based on the at least one area of anatomic constraint, the ablation treatment plan including a treatment order for the passageways and a plurality of sequential treatment locations.

14. The non-transitory machine-readable media of claim 13, wherein the plurality of sequential treatment locations includes a start treatment location at which a first ablation treatment of the ablation treatment plan is delivered and a stop treatment location at which a final ablation treatment of the ablation treatment plan is delivered.

15. The non-transitory machine-readable media of claim 13, wherein the area of anatomic constraint includes an obstruction in a passageway of the plurality of passageways.
